# EUROPÄISCHE PATENTSCHRIFT

(11) **EP 0 443 666 B1**
(45) Veröffentlichungstag und Bekanntmachung des Hinweises auf die Patenterteilung: **10.05.1995**
(21) Anmeldenummer: 91200298.7
(22) Anmeldetag: 13.02.1991
(51) Int. Cl.: A61B 6/02, H05G 1/64

(54) **Röntgendiagnostikgerät mit Mitteln zur vergrösserten visuellen Darstellung eines wählbaren Ausschnitts des Gesamt-Bildbereichs**
X-ray diagnostic apparatus with means for an enlarged visual presentation of a selectable sector of the total image area
Appareil diagnostic à rayons-X comportant des moyens pour la présentation visuelle agrandie d'un secteur de l'image globale

(30) Priorität: 17.02.1990 DE 4005111
(43) Veröffentlichungstag der Anmeldung: 28.08.1991
(73) Patentinhaber: Philips Patentverwaltung GmbH, 22335 Hamburg (DE); Philips Electronics N.V., 5621 BA Eindhoven (NL)
(72) Erfinder: Haaker, Paul, W-2000 Hamburg 61 (DE); Klotz, Erhard, W-2211 Hodorf (DE); Koppe, Reiner, Dr., W-2000 Hamburg 61 (DE); Linde, Rolf, W-2081 Haseldorf (DE)
(74) Vertreter: Hartmann, Heinrich, Dipl.-Ing.

(56) Entgegenhaltungen:
- EP-A- 0 083 465
- EP-A- 0 088 356
- DE-C- 1 070 402
- GB-A- 2 017 450

## Beschreibung

Die Erfindung bezieht sich auf ein Röntgendiagnostikgerät mit einer Röntgenstrahlungsquelle, mit einer die Intensitätsverteilung der durch ein Prüfobjekt richtbaren Röntgenstrahlung darstellenden Abbildungsfläche, mit einer optischen Abbildungseinrichtung zur Abbildung eines Gesamt-Bildbereichs der Abbildungsfläche auf der Eingangsfläche eines ersten Bildsensors, mit einer Einrichtung zur visuellen Darstellung des Gesamtbildbereichs und mit Mitteln zur vergrößerten visuellen Darstellung eines wählbaren Ausschnitts des Gesamt-Bildbereichs.

Bei einer durch die DE-OS 33 19 309 bekannten derartigen Einrichtung ist ein Fernsehaufnahmesystem vorgesehen, mit welchem mittels elektronischer Maßnahmen ein wählbarer Bildausschnitt auf einem Monitor darstellbar ist. Dabei werden Abtastgeschwindigkeit, Horizontalfrequenz und Vertikalfrequenz derart verändert, daß ein Bildausschnitt mit erhöhter Bildpunktzahl pro Flächeneinheit abgetastet wird. Eine Erhöhung der Bildpunktzahl pro Flächeneinheit ist bei einer Bildaufnahmeröhre (Vidicon, Plumbicon) nur in begrenztem Umfang möglich, so daß mit zunehmender Ausschnittsvergrößerung eine größere Unschärfe des Ausschnittbildes unvermeidbar ist.

Der Erfindung liegt die Aufgabe zugrunde, eine Röntgendiagnostikeinrichtung der eingangs genannten Art derart zu gestalten, daß auf einfache Weise detailauflösende, stark vergrößerte Ausschnittsbilder erzeugbar sind.

Die Lösung gelingt dadurch, daß der wählbare Ausschnitt des Gesamt-Bildbereichs auf einen zweiten Bildsensor gerichtet ist, welcher in bezug auf die Flächeneinheit des Gesamt-Bildbereichs eine wesentlich höhere Bildpunktzahl diskriminiert.

Zur Beobachtung des Gesamt-Bildbereichs ist beispielsweise mit einer Bildpunktzahl von 512 x 512 eine ausreichend scharf erscheinende Auflösung möglich. Wenn die gleiche Anzeigefläche mit einem vergrößerten Ausschnitt möglichst vollständig ausgefüllt werden soll, kann man den zweiten Bildsensor erfindungsgemäß unabhängig vom ersten derart auslegen, daß der Ausschnitt mit zumindest annähernd gleicher Bildpunktzahl und damit gleich scharf dargestellt wird.

Als ersten Bildsensor wählt man in vorteilhaft bewährter Weise eine TV-Kamera mit z.B. Vidicon- oder Plumbiconröhre.

Damit der Bildausschnitt durch den zweiten Bildsensor schnell und einfach erhalten werden kann, ist in Weiterbildung der Erfindung vorgesehen, daß auf den zweiten Bildsensor ein abgezweigter Teil der in die optische Abbildungseinrichtung gelangenden Strahlung gerichtet ist.

Obgleich prinzipiell beliebige bekannte Bildsensoren verwendbar sind, wird vorzugsweise als zweiter Bildsensor ein CCD-Bildsensor verwendet, welcher auf kleiner Fläche eine hohe Bildpunktzahl scharf detektieren kann. Derartige, für den Röntgendurchleuchtungsbetrieb geeignete CCD-Bildsensoren sind in Radiol. diagn. 25 (1984) Seiten 799-803 beschrieben.

Die Abzweigung eines Teils der Strahlung der optischen Abbildungseinrichtung ist nicht erforderlich, wenn CCD-Sensoren verwendet werden, welche direkt Röntgenstrahlung detektieren und hinter dem Prüfobjekt in den Weg der Röntgenstrahlung bringbar sind. Für diesen Zweck ist vorteilhaft ein Lumineszenz-Detektor (stimulierbarer Phosphor) geeignet, dessen Abbildungsfläche von einem Laserstrahl abgetastet wird.

Gemäß einer bevorzugten Lösung ist vorgesehen, daß die Eingangsfläche des zweiten Bildsensors derart gering bemessen ist, daß nur ein Ausschnitt der Gesamt-Bildfläche erfaßbar ist, und daß der zweite Bildsensor in zwei Koordinatenrichtungen über den der Gesamtbildfläche entsprechenden Bereich verschiebbar ist. Dann ergeben sich kleinflächige Bildsensoren, welche wegen ihrer geringen Größe und Masse mit geringem Aufwand mechanisch in zwei Koordinatenrichtungen in den Bereich der Gesamt-Bildfläche verschiebbar sind, welcher detaillierter untersucht werden soll.

Damit die insbesondere für medizinische Anwendungen sich ergebende strahlenbelastung für den Patienten möglichst gering bleibt, ist vorgesehen, daß der Röntgenstrahlungsquelle eine Blende nachgeordnet ist, welche ein dem vom zweiten Bildsensor erfaßten Ausschnitt entsprechendes Röntgenstrahlfeld durchläßt. Ein weiterer bedeutsamer Vorteil dieser Lösung ist, daß dabei die Streustrahlung minimiert wird, so daß der Bildkontrast erhöht und eine weitere Reduzierung der Strahlendosis ermöglicht wird.

Zur Vereinfachung des Einstellaufwandes ist vorgesehen, daß die Koordinatenbewegungen des zweiten Bildsensors und der Blende durch eine gemeinsame Steuerschaltung veranlaßt sind.

Eine bevorzugte Lösung ist dadurch gekennzeichnet, daß die Koordinatenbewegungen durch eine Cursorschaltung gesteuert sind, und daß das Bewegungsziel durch einen auf die Mitte des zu vergrößernden Ausschnitts des visuell dargestellten Gesamt-Bildbereichs gerichteten Zielstift vorbestimmt ist. Dann ergibt sich ein einfach bedienbares Röntgendiagnostikgerät, bei welchem anhand zuvor mittels des ersten Sensors beobachteter Übersichtsaufnahmen an beliebigen Stellen erheblich vergrößerte Ausschnittsbilder erzeugt werden können.

Bei Aufnahme sich periodisch bewegender Prüfobjekte ist es in Weiterbildung der Erfindung wichtig, daß aufeinanderfolgende Aufnahmen durch den Bewegungsablauf des Prüfobjekts, beispielsweise eines menschlichen Herzens, synchronisiert sind.

Die Erfindung ermöglicht ohne größere bauliche Veränderungen bestehender Röntgendiagnostikeinrichtungen eine sehr viel genauere geometrische Vermessung feiner Strukturen und ist insbesondere für medizinische Anwendungen geeignet. Eine bessere Kenntnis über den morphologischen Zustand von Gefäßen erleichtert insbesondere die vielfältige Behandlungsmöglichkeit von Stenosen (Gefäßverengungen).

Die Erfindung wird anhand der Beschreibung eines in der Zeichnung dargestellten bevorzugten Ausführungsbeispiels näher erläutert.

In der Figur ist die Systemanordnung einer erfindungsgemäß mit einem zweiten Bildsensor ausgestatteten Röntgendiagnostikeinrichtung dargestellt.

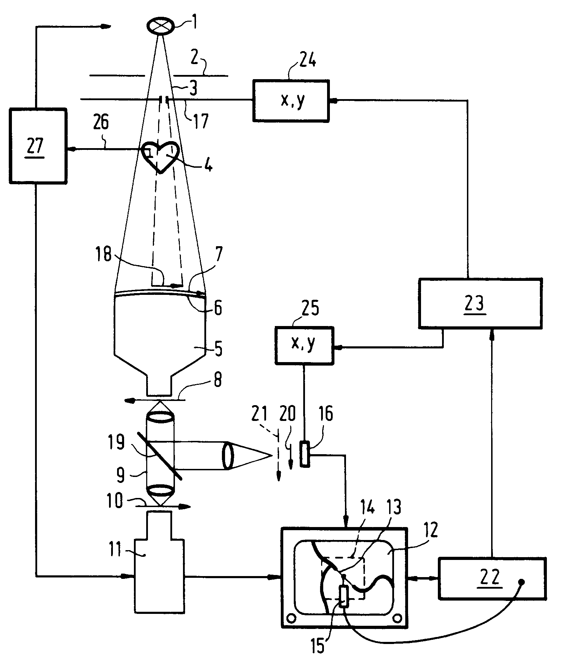

Mit 1 ist eine Röntgenstrahlenquelle angedeutet, welche in bekannter Weise ausgebildet sein kann. Diese sendet ein durch eine Blende 2 begrenztes Röntgenstrahlbündel in Richtung auf ein Untersuchungsobjekt, beispielsweise ein Herz 4. Die das untersuchungsobjekt durchdringende Strahlung bildet auf der Abbildungsfläche 6 des Röntgenbildverstärkers 5 ein Schattenbild 7. Am Ausgang des Röntgenbildverstärkers 5 steht ein verstärktes Bild 8 zur Verfügung, welches über die optische Abbildungseinrichtung 9 als Gesamt-Bildbereich 10 vom Target einer TV-Kamera 11 detektiert und auf dem Bildschirm 12 visuell dargestellt wird. Es ist eine Stenose 13 erkennbar, welche genauer betrachtet-werden soll, indem der gestrichelt angedeutete Bereich 14 so weit vergrößert wird, daß er die gesamte Bildschirmfläche ausfüllt.

Die Bedienungsperson gibt die Lage des Ausschnitts mittels eines auf die Mitte des Bereichs 14 gerichteten Zielstiftes 15 vor. Danach wird vom zweiten CCD-Bildsensor 16 das gewünschte Ausschnittsbild detektiert und in ein Videosignal umgewandelt, welches eine vollflächige Darstellung auf dem Bildschirm 12 ermöglicht.

Zur Verringerung der Strahlenbelastung insbesondere eines Patienten und zur Verringerung von kontrastmindernder Streustrahlung ist eine Blende 17 vorgesehen, welche nur ein auf den Ausschnittsbereich verengtes Röntgenstrahlenbündel durchläßt, so daß auf der Abbildungsfläche 6 des Röntgenbildverstärkers nur ein entsprechend kleineres Schattenbild 18 entsteht, welches über den halbdurchlässigen Spiegel 19 der optischen Abbildungseinrichtung 9 als Ausschnittsbild 20 die Eingangsfläche des zweiten CCD-Bildsensors 16 ausfüllt. Ohne Blende 17 würde an dieser Stelle ein erheblich größeres Gesamtbild 21 verfügbar sein, welches dem Gesamt-Bildbereich 10 entspricht.

In Abhängigkeit der Befehlssignale der Cursorschaltung 22 veranlaßt die Steuerschaltung 23 Bewegungen der Manipulatoren 24 und 25 in Koordinatenrichtungen x und y, so daß die Blende 17 und der CCD-Bildsensor 16 in die vom Zielstift 15 vorgegebene Zielposition verfahren werden.

Damit aufeinanderfolgende Aufnahmen wie Gesamtaufnahme und Ausschnittsaufnahme stets in gleicher Phasenlage der rythmischen Herzbewegung erfolgen, ist eine von einem EKG-Signal 26 getriggerte Synchronisiersteuerung 27 vorgesehen, welche die jeweils phasengleiche Aktivierung der Röntgenstrahlungsquelle 1 und der Bildsensoren 11 und 16 bewirkt. Selbstverständlich können Speicherschaltungen vorgesehen werden, welche die jeweils detektierten Bilddaten der Sensoren 11 und 16 speichern.

Der Gesamt-Bildbereich 10 wird auf dem Bildschirm 12 mit einer Bildpunktzahl von beispielsweise 512 x 512 dargestellt. Das beispielsweise 6-fach zu vergrößernde Ausschnittsbild 20 wird vom CCD-Sensor 16 mit einer Bildpunktzahl von 300 x 400 detektiert. Dieser CCD-Sensor 16 erfordert wegen seiner geringen Abmessungen von etwa 1,5 x 1 cm² nur geringen konstruktiven Aufwand zur Verschiebung mittels des Manipulators. Entsprechendes gilt für den Manipulator 24.

Die Bildpunktdichte, bezogen auf die Flächeneinheit des Gesamt-Bildbereichs 10 bzw. 20 ist dann für das auf dem Bildschirm 12 dargestellte Ausschnittsbild 20 vervielfacht, so daß Detailstrukturen der Stenose 13 genauer bestimmt werden können.

## Patentansprüche

1. Röntgendiagnostikgerät mit einer Röntgenstrahlungsquelle (1), mit einer die Intensitätsverteilung der durch ein Prüfobjekt (4) richtbaren Röntgenstrahlung darstellenden Abbildungsfläche (6), mit einer optischen Abbildungseinrichtung zur Abbildung eines Gesamt-Bildbereichs (10) der Abbildungsfläche (6) auf der Eingangsfläche eines ersten Bildsensors (11), mit einer Einrichtung (12) zur visuellen Darstellung des Gesamtbildbereichs (10) und mit Mitteln zur vergrößerten visuellen Darstellung eines wählbaren Ausschnitts (20) des Gesamt-Bildbereichs,
dadurch gekennzeichnet, daß der wählbare Ausschnitt (20) des Gesamt-Bildbereichs auf einen zweiten Bildsensor (16) gerichtet ist, welcher in bezug auf die Flächeneinheit des Gesamt-Bildbereichs eine wesentlich höhere Bildpunktzahl diskriminiert.

2. Röntgendiagnostikgerät nach Anspruch 1,
dadurch gekennzeichnet, daß der erste Bildsensor eine Videokamera (11) ist.

3. Röntgendiagnostikgerät nach Anspruch 1 oder 2,
dadurch gekennzeichnet, daß auf den zweiten Bildsensor ein abgezweigter Teil der in die optische Abbildungseinrichtung (9) gelangenden Strahlung gerichtet ist.

4. Röntgendiagnostikgerät nach einem der Ansprüche 1 bis 3,
dadurch gekennzeichnet, daß der zweite Bildsensor ein CCD-Bildsensor (16) ist.

5. Röntgendiagnostikgerät nach einem der Ansprüche 1 bis 3,
dadurch gekennzeichnet, daß der zweite Bildsensor ein Röntgenstrahlung detektierender CCD-Sensor ist, welcher hinter dem Prüfobjekt in den Weg der Röntgenstrahlung bringbar ist.

6. Röntgendiagnostikgerät nach Anspruch 5,
dadurch gekennzeichnet, daß der zweite Bildsensor ein Lumineszenz-Detektor ist, dessen Abbildungsfläche von einem Laserstrahl abgetastet wird.

7. Röntgendiagnostikeinrichtung nach einem der Ansprüche 1 bis 6,
dadurch gekennzeichnet, daß die Eingangsfläche des zweiten Bildsensors (16) derart gering bemessen ist, daß nur ein Ausschnitt der Gesamt-Bildfläche (21) erfaßbar ist, und daß der zweite Bildsensor (16) in zwei Koordinatenrichtungen (x,y) über den der Gesamtbildfläche (21) entsprechenden Bereich verschiebbar ist.

8. Röntgendiagnostikeinrichtung nach Anspruch 7,
dadurch gekennzeichnet, daß der Röntgenstrahlungsquelle eine Blende (17) nachgeordnet ist, welche ein dem vom zweiten Bildsensor (16) erfaßten Ausschnitt (18) entsprechendes Röntgenstrahlfeld durchläßt.

9. Röntgenstrahlenquelle nach Anspruch 7 oder 8,
dadurch gekennzeichnet, daß die Koordinatenbewegungen des zweiten Bildsensors (16) und der Blende (17) durch eine gemeinsame Steuerschaltung (2) veranlaßt sind.

10. Röntgendiagnostikgerät nach einem der Ansprüche 7 bis 9,
dadurch gekennzeichnet, daß die Koordinatenbewegungen durch eine Cursorschaltung (22) gesteuert sind, und daß das Bewegungsziel durch einen auf die Mitte des zu vergrößernden Ausschnitts (14) des visuell dargestellten Gesamtbildbereichs (12) gerichteten Zielstift (15) vorbestimmt ist.

11. Röntgendiagnostikgerät nach einem der Ansprüche 1 bis 10,
dadurch gekennzeichnet, daß die Röntgenstrahlimpulse im Falle der Untersuchung eines sich periodisch bewegenden Prüfobjekts (4) durch den Bewegungsablauf synchronisiert sind.

## Claims

1. An X-ray diagnostic apparatus, comprising an X-ray source (1), an imaging surface (6) for forming an image of the intensity distribution of the X-rays to be directed through a test object (4), an optical imaging device for imaging an overall image area (10) of the imaging surface (6) onto the entrance surface of a first image sensor (11), a device (12) for visual display of the overall image area (10), and means for the enlarged visual display of a selectable part (20) of the overall image area, characterized in that the selectable part (20) of the overall image area is directed onto a second image sensor (16) which discriminates a substantially higher number of pixels with respect to the unit of surface area of the overall image area.

2. An X-ray diagnostic apparatus as claimed in Claim 1, characterized in that the first image sensor is a video camera (11).

3. An X-ray diagnostic apparatus as claimed in Claim 1 or 2, characterized in that a part of the radiation entering the optical imaging device (9) is branched off so as to be directed onto the second image sensor.

4. An X-ray diagnostic apparatus as claimed in any one of the Claims 1 to 3, characterized in that the second image sensor is a CCD image sensor (16).

5. An X-ray diagnostic apparatus as claimed in any one of the Claims 1 to 3, characterized in that the second image sensor is a CCD sensor which detects X-rays and can be inserted into the path of the X-rays behind the object to be tested.

6. An X-ray diagnostic apparatus as claimed in Claim 5, characterized in that the second image sensor is a luminescence detector whose imaging surface is scanned by a laser beam.

7. An X-ray diagnostic apparatus as claimed in any one of the Claims 1 to 6, characterized in that the entrance surface of the second image sensor (16) is proportioned to be so small that only a part of the overall image area (21) can be detected, the second image sensor (16) being displaceable across the area corresponding to the overall image area (21) in two coordinate directions (x, y).

8. An X-ray diagnostic apparatus as claimed in Claim 7, characterized in that the X-ray source is succeeded by a diaphragm (17) which transmits an X-ray field corresponding to the part (18) detected by the second image sensor (16).

9. An X-ray diagnostic apparatus as claimed in Claim 7 or 8, characterized in that the coordinate movements of the second image sensor (16) and the diaphragm (17) are controlled by a common control circuit (2).

10. An X-ray diagnostic apparatus as claimed in any one of the Claims 7 to 9, characterized in that the coordinate movements are controlled by a cursor circuit (22), the target being predetermined by a point-out pen (15) aimed at the centre of the area (14) to be enlarged of the visually displayed overall image area (12).

11. An X-ray diagnostic apparatus as claimed in any one of the Claims 1 to 10, characterized in that in the case of examination of a periodically moving test object (4), the X-ray pulses are synchronized with the motion.

## Revendications

1. Appareil de diagnostic à rayons X, comportant une source de rayons X (1), une surface de formation d'image (6) représentant la distribution d'intensité des rayons X dirigés à travers un objet à examiner (4), un dispositif imageur optique pour présenter une image d'une zone d'image globale (10) de la surface de formation d'image (6) sur la surface d'entrée d'un premier capteur d'image (11), un dispositif (12) pour l'affichage vidéo de la zone d'image globale (10) et des moyens pour l'affichage vidéo agrandi d'un secteur sélectionnable (20) de la zone d'image globale, caractérisé en ce que le secteur sélectionnable (20) de la zone d'image globale est dirigé sur un deuxième capteur d'image (16), qui discrimine un nombre de points d'image sensiblement plus élevé par rapport à l'unité de surface de la zone d'image globale.

2. Appareil de diagnostic à rayons X selon la revendication 1, caractérisé en ce que le premier capteur d'image est une caméra vidéo (11).

3. Appareil de diagnostic à rayons X selon la revendication 1 ou 2, caractérisé en ce qu'une partie déviée des rayons arrivant sur le dispositif imageur optique (9) est dirigée sur le deuxième capteur d'image.

4. Appareil de diagnostic à rayons X selon l'une quelconque des revendication 1 à 3, caractérisé en ce que le deuxième capteur d'image est un capteur d'image CCD (16).

5. Appareil de diagnostic à rayons X selon l'une quelconque des revendications 1 à 3, caractérisé en ce que le deuxième capteur d'image est un capteur CCD détectant les rayons X, qui peut être placé derrière l'objet à examiner dans le chemin des rayons X.

6. Appareil de diagnostic à rayons X selon la revendication 5, caractérisé en ce que le deuxième capteur d'image est un capteur de luminescence, dont la surface de formation d'image est balayée par un faisceau laser.

7. Dispositif de diagnostic à rayons X selon l'une quelconque des revendications 1 à 6, caractérisé en ce que la surface d'entrée du deuxième capteur d'image (16) a des dimensions assez petites pour que seul un secteur de la surface d'image globale (21) puisse être capté et que le deuxième capteur d'image (16) peut être déplacé dans deux directions de coordonnées (x, y) sur la zone correspondant à la surface d'image globale (21).

8. Dispositif de diagnostic à rayons X selon la revendication 7, caractérisé en ce que l'on agence en aval de la source de rayons X un diaphragme (17) qui laisse passer un champ de rayons X correspondant au secteur (18) capté par le deuxième capteur d'image (16).

9. Dispositif de diagnostic à rayons X selon la revendication 7 ou 8, caractérisé en ce que les déplacements selon des axes de coordonnées du deuxième capteur d'image (16) et du diaphragme (17) sont effectués par un circuit de commande commun (2).

10. Appareil de diagnostic à rayons X selon l'une quelconque des revendications 7 à 9, caractérisé en ce que les déplacements selon des axes de coordonnées sont pilotés par un circuit à curseur (22) et la cible de déplacement est prédéterminée par un photostyle (15) dirigé sur la partie centrale du secteur à agrandir (14) de la zone d'image globale affichée (12).

11. Appareil de diagnostic à rayons X selon l'une quelconque des revendications 1 à 10, caractérisé en ce que les impulsions du faisceau de rayons X sont synchronisées par le mouvement, dans le cas de l'examen d'un objet (4), qui effectue un mouvement périodique.
